# EUROPEAN PATENT SPECIFICATION

(11) **EP 2 303 163 B1**
(45) Date of publication and mention of the grant of the patent: **23.11.2011**
(21) Application number: 09749883.6
(22) Date of filing: 20.05.2009
(51) Int. Cl.: A61B 17/70

(54) **SYSTEM FOR STABILIZING AT LEAST THREE VERTEBRAE**
SYSTEM ZUR STABILISIERUNG VON MINDESTENS DREI WIRBELN
SYSTÈME DE STABILISATION D'AU MOINS TROIS VERTÈBRES

(30) Priority: 20.05.2008 EP 08305183
(43) Date of publication of application: 06.04.2011
(73) Proprietor: ZIMMER SPINE, 33000 Bordeaux (FR)
(72) Inventor: BELLIARD, Karl P., F-49770 La Membrolle Sur Longuenee (FR)
(74) Representative: Besnard, Christophe Laurent
(86) International application number: PCT/EP2009/056168
(87) International publication number: WO 2009/141393

(56) References cited:
- FR-A- 2 722 088
- US-A1- 2006 235 387
- US-A1- 2008 033 557

## Description

### FIELD OF THE INVENTION

The present disclosure relates to a system for stabilizing at least three vertebrae, i.e. a system that holds together at least three vertebrae. More particularly, it relates to a system that, while holding together the vertebrae, nevertheless allows a limited amount of relative movement between them, thereby providing what is called a "dynamic stabilization".

### BACKGROUND OF THE INVENTION

When it is desired to stabilize only two vertebrae, it is known to place between them an intervertebral implant constituted by a spacer that is usually disposed between the two spinous processes of the vertebrae, together with a braid or a tie that surrounds the spinous processes in order to hold the spacer between the vertebrae.

For instance, it is known to use a tie that presents two free end portions that are secured to the spacer with the help of a self-locking system of the type described in PCT patent application WO 2005/120277.

It is also known from US patent application US 2006/0235387, to use one spacer and two ties, each tie being inserted through a central conduit made in the spacer and passing around the transverse process of one of the two vertebra, the two free end portions of the tie being tied together by means of a stopper.

Document US2008033557 A1 discloses another vertebral stabilization system.

However, with certain pathologies of the vertebral column, the surgeon might need to perform surgery for stabilizing more than two vertebrae, that is to say securing at least three vertebrae to each other (e.g. three or four vertebrae).

### SUMMARY OF THE INVENTION

A first object of the present disclosure is to provide a stabilization system for stabilizing at least three vertebrae.

Another object of the present disclosure is to provide a stabilization system that enables the surgeon to exert the required amount of traction on the free end portions of the tie system under improved conditions, and regardless of the number of vertebrae that are to be stabilized.

To achieve these objects, according to one aspect of the present disclosure, there is provided a stabilization system for stabilizing at least three vertebrae, comprising:
- a single flexible tie having two free end portions and intermediate portions therebetween, said tie being adapted to form a single loop around the spinous processes of said (at least three) vertebrae; and
- a group of at least two spacers, each spacer being adapted to be interposed between the spinous processes of two adjacent vertebrae, said group of spacers comprising at least:
- a first spacer having guiding means for guiding two intermediate portions of the tie; and
- a second spacer with a tie-securing system through which the two free end portions of the tie can be inserted and pulled, the tie-securing system allowing said free end portions to be fixedly secured to the second spacer.

Such a stabilization system, while having a quite limited number of parts and a simple construction, can be used for holding together three or more vertebrae.

Since said tie forms a single loop around the spinous processes of the three or more vertebrae to be stabilized, and since there is only two free end portions suitable for exerting traction, the surgeon can easily exert said traction.

Thanks to said guiding means and securing system, each part of the stabilization system cooperates with another part of the system, thereby allowing the surgeon to easily and correctly position these parts with respect to each other.

The tie-securing system, which is secured to the second spacer, may form an integral portion of the spacer or may be removably mounted thereon.

In an embodiment, the first spacer does not have any tie-securing system.

In an embodiment, in addition to its tie-securing system, the second spacer has guiding means for guiding one intermediate portion of the tie.

In another embodiment, the first spacer has a tie-securing system. But, in such a case, the tie-securing system of the first spacer is not used for securing the two free end portions of the tie, but for guiding one intermediate portion of the tie. Thus, in this case, the tie-securing system of the first spacer forms guiding means for guiding a first intermediate portion of the tie, and the first spacer further comprises other guiding means for guiding a second intermediate portion of the tie.

In another embodiment, the first spacer and the second spacer are similar, both comprising a tie-securing system and guiding means for guiding one intermediate portion of the tie. In such a case, the tie-securing system of the first spacer is to be considered, functionally, as guiding means for guiding one intermediate portion of the tie, as explained above.

According to another aspect of the present disclosure, there is provided a system for stabilizing at least three vertebrae, comprising:
- at least two flexible ties adapted to form together a single loop around the spinous processes of said (at least three) vertebrae, each tie having two free end portions and intermediate portions therebetween; and
- at least two spacers, each spacer being adapted to be interposed between the spinous processes of two adjacent vertebrae, and each spacer comprising:
- guiding means for guiding an intermediate portion of one of said ties; and
- a tie-securing system through which one free end portion of each of said two ties can be inserted and pulled, the tie-securing system allowing these two free end portions to be fixedly secured to the spacer.

Such a stabilization system, while having a quite limited number of parts and a simple construction, can be used for holding together three or more vertebrae.

Since said two ties form together a single loop around the spinous processes of the three or more vertebrae to be stabilized, even if there are, physically, two pairs of free end portions, there are, functionally, only two free end portions suitable for exerting traction and the surgeon can easily exert said traction. The surgeon can even select the pair of free end portions that is easier to access.

Thanks to said guiding means and securing system, each part of the stabilization system cooperates with another part of the system, thereby allowing the surgeon to easily and correctly position these parts with respect to each other.

The tie-securing system, which is secured to each spacer, may form an integral portion of the spacer or may be removably mounted thereon.

In all of the above-defined embodiments, regardless of the number of ties or braids that are used, each tie may be made of a material that presents a certain amount of elasticity, so that even after the surgeon has pulled and locked the free end portions of the tie(s), each tie allows a limited amount of relative movement to the three or more vertebrae while providing a stabilizing effect, thereby providing a dynamic stabilization.

The above-mentioned features and advantages of the present disclosure, as well as others, are described in detail below. The following detailed description is exemplary and explanatory only, and is not restrictive of the invention as claimed. The detailed description refers to the accompanying drawings.

### BRIEF DESCRIPTION OF THE DRAWINGS

In the drawings, like reference signs generally refer to the same parts throughout the different views.
Figure 1 is a general view of an example of system for stabilizing three vertebrae, having a simple spacer and a spacer provided with a tie-securing system;
Figure 2 is an general view of an example of system for stabilizing four vertebrae, using two simple spacers and one spacer provided with a tie-securing system;
Figure 3 is a perspective view of a spacer provided with a first type of tie-securing system;
Figure 4 is a view of the underside of the Figure 3 spacer;
Figure 5 is a longitudinal section view of the Figure 4 spacer on the plane A-A of Figure 4;
Figure 6 is a perspective view of a spacer fitted with a second type of tie-securing system;
Figures 7, 8, and 9 show the various component parts of the spacer shown in Figure 6;
Figure 10 is a perspective view of a spacer fitted with a third type of tie-securing means;
Figure 11 is an exploded view of the spacer shown in Figure 10;
Figure 12 is a longitudinal section view on plane C-C of the Figure 11 spacer;
Figure 13 is a perspective view of a spacer provided with a fourth type of tie-securing system;
Figure 14 is a section view on plane B-B of Figure 13;
Figure 15 is a perspective view of a spacer provided with a fifth type of tie-securing system;
Figure 16 is a longitudinal section view of the Figure 15 spacer;
Figure 17 is a cross-section view on plane A-A of Figure 16;
Figure 18 is a general view of an example of stabilization system for four vertebrae using another type of spacer fitted with a sixth type of tie-securing system;
Figures 19 and 20 are perspective views of the spacer used in the stabilization system of Figure 18;
Figure 21 is a longitudinal section view of the tie-securing system of the spacer shown in Figures 19 and 20;
Figure 22 is a general view showing another example of system for stabilizing three vertebrae;
Figure 23 is a general view showing another example of system for stabilizing four vertebrae;
Figure 24 shows a variant of the Figure 22 stabilization system;
Figures 25 and 26 show two other examples of stabilization system;
Figure 27 shows an example of a simple spacer.

### DETAILED DESCRIPTION OF THE INVENTION

In all the accompanying drawings, vertebrae carry the reference signs V1, V2, V3, V4, and the spinous processes of these vertebrae V1, V2, V3, V4 are diagrammatically shown in cross-section by hatched oval shapes.

In the present disclosure, a spacer is qualified as "simple", when it is not fitted with a tie-securing system.

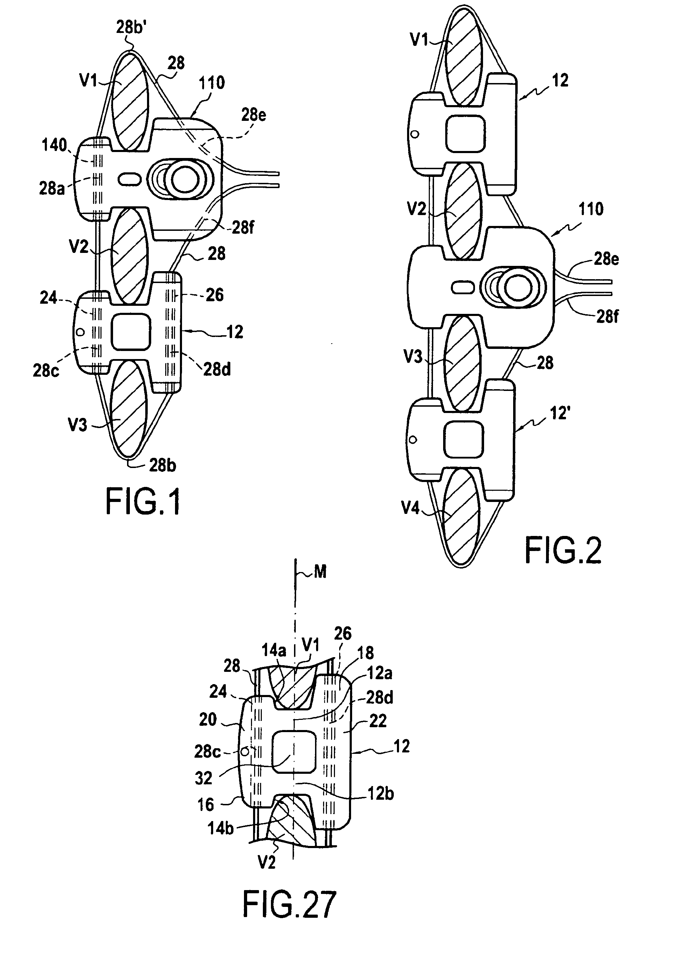

More precisely, an example of simple spacer is shown on Figure 27. This spacer 12 has upper and lower parts 12a and 12b provided, on their end faces (i.e. the upper and lower end faces of the spacer 12), with recesses 14a and 14b for receiving, respectively, the two spinous processes of two adjacent vertebrae V1 and V2.

Each recess 14a, 14b, is defined between two prongs: a distal prong 16 (on the left in figure 27) and a proximal prong 18 (on the right in figure 27). Thus, there are on the opposed lateral sides (i.e. left and right sides) of the spacer 12, respectively, a pair of distal prongs 16 and a pair of proximal prongs 18. The distal prongs 16 are much smaller in height than are the proximal prongs 18. As is known, this asymmetry of the prongs makes it possible to use a lateral approach for putting the spacer 12 into place between the spinous processes of vertebrae V1 and V2 (see PCT patent application WO 2007/099258).

The lateral portions 20 and 22 of the spacer that terminate in the prongs 16 and 18 are provided with internal passages 24, 26 that extend over their full length so as to allow a securing braid or tie 28 to pass freely. The two passages 24, 26, are substantially parallel and located on either side of the median plane M of the spacer 12 (the median plane M of the spacer 12 corresponds to the median plane of the spine, when the spacer is in place). Each passage 24, 26 opens out into the upper and lower end faces of the spacer 12. The two passages 24, 26, form together guiding means for guiding two intermediate portions 28c, 28d, of a tie 28, each passage guiding one intermediate portion. Therefore, the simple spacer 12 acts relative to the tie 28 solely to provide guidance, which is achieved by the internal passages 24, 26.

It should be understood that the structure of the simple spacer 12 is very simple, since it does not itself include any tie-securing system.

Figure 1 shows an embodiment of stabilization system which involves stabilizing three adjacent vertebrae V1, V2, and V3.

Such a stabilization system comprises:
- a tie 28;
- a simple spacer 12, located between vertebrae V2 and V3, similar to the simple spacer of Figure 27 described above; and
- a spacer 110, located between vertebrae V1 and V2, having a system for clamping and securing the free end portions of the tie 28.

The tie 28 is made of a material that confers a certain amount of flexibility thereto and possibly also a certain amount of elasticity, in order to obtain the desired dynamic stabilization effect.

The tie 28 has two free end portions 28e, 28f, and intermediate portions 28a, 28b, 28b', 28c, 28d, therebetween. Tie 28 is adapted to form a single loop around the spinous processes of vertebrae V1, V2, V3, and two intermediate portions 28b, 28b' of the tie respectively rest on the spinous processes of the end vertebrae V1 and V3. Other intermediate portions 28c, 28d of the tie 28 are engaged in the internal passages 24, 26 of spacer 12. Each intermediate portion 28c, 28d passes freely along one of said internal passage 24, 26.

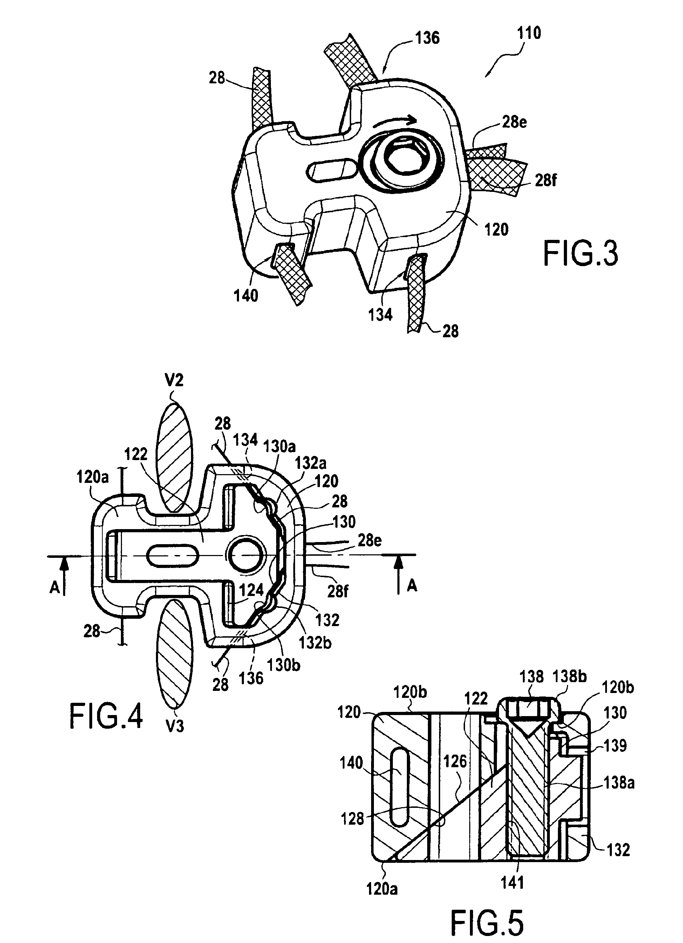

With reference now to Figures 3, 4 and 5, there follows a description of the spacer 110 incorporating a tie-securing system.

In this embodiment, the system for securing the free end portions 28e, 28f of the tie 28, also called locking system, forms an integral portion of the spacer 110. This locking system is constituted by a stationary part, formed by the body 120 of the spacer 110, and by a moving part 122.

The moving part 122 is mounted in a housing 124, or cavity, made in the body 120 and opening out into its bottom face 120a (see figure 4). The moving element 122 presents a sloping top face 126 that co-operates with the sloping bottom face 128 of the housing 124, as shown on figure 5. By the face 126 sliding against the face 128, the end 130 of the moving part 122 can be moved towards the front wall 132 of the housing 124. The spacer body 120 also has two slots 134 and 136 for passing the tie 28 that can thus penetrate into the inside of the housing 124. The front face 130 of the moving part 122 defines two clamping surfaces 130a and 130b that co-operate with clamping surfaces 132a and 132b defined by the front wall 132 of the housing 124. Since the tie 28 is disposed between these two pairs of clamping surfaces, moving the part 122 towards the front wall 132 serves to clamp the tie 28.

In Figure 5, it can be seen that the front wall 132 of the housing 124 is provided with a slot 139 that is located between the clamping surfaces 132a and 132b, that opens out outside, and that enables the free end portions 28e and 28f of the tie 28 to pass through after they have been passed between the clamping surfaces 130a, 132a, 130b, 132b.

The moving part 122 is preferably moved relative to the stationary body 120 with the help of a screw 138 having its threaded portion 138a co-operating with a tapped bore 141 formed in the moving part 122. In addition, the head 138b of the screw 138 is held pressed against the top face 120b of the body 120 of the spacer 110. Referring to Figure 5, it will be understood that by turning the screw 138, it moves in the up and down direction and, thereby, drives the moving part 122 in the left and right direction, i.e. the moving part 122 moves apart from, or comes closer to, the front wall 132 of the body 120.

On the opposite side of the front wall 132 (with respect to the median plane of the spacer), the spacer body 120 has an internal passage 140 extending through its thickness, being suitable for receiving an intermediate portion 28a of the tie 28 (see Figures 1 and 5) and through which the intermediate portion 28a can slide freely.

It is to be noted that, as the simple spacer 12, the spacer 110 has two opposite recesses for receiving the spinous processes of the two adjacent vertebrae V1 and V2. Each recess is defined between two prongs: a distal prong (on the left on figure 1) and a proximal prong (on the right on figure 1). The distal prongs are smaller in height than are the proximal prongs, so that the transverse height of the distal end of the spacer 110 is smaller, while the transverse height of the proximal end of the spacer 110 is larger (since the proximal end of the spacer 110 does not need to be capable of being inserted between the vertebrae V1 and V2). Such an asymmetry of the spacer 110 makes it possible to use a lateral approach for putting the spacer 110 into place between the spinous processes of vertebrae V1 and V2.

Figure 2 shows another embodiment of stabilization system which involves stabilizing four adjacent vertebrae V1, V2, V3 and V4.

Such a stabilization system comprises:
- a tie 28;
- two simple spacers 12, 12', as described above.
- a spacer 110 having a system for clamping and securing the free end portions 28e, 28f of the tie 28, as described above.

The spacer 12 is located between adjacent vertebrae V1 and V2. The spacer 110 is located between adjacent vertebrae V2 and V3. The spacer 12' is located between adjacent vertebrae V3 and V4. The simple spacer 12 is identical to the simple spacer 12'.

As for the embodiment of Figure 1, with the embodiment of Figure 2 it is possible to insert the intervertebral spacers 12, 12', 110 using a lateral approach and it is possible to exert traction laterally on the ends of the tie 28. In these embodiments the simple spacers 12 and 12' merely perform a function of guiding the tie 28.

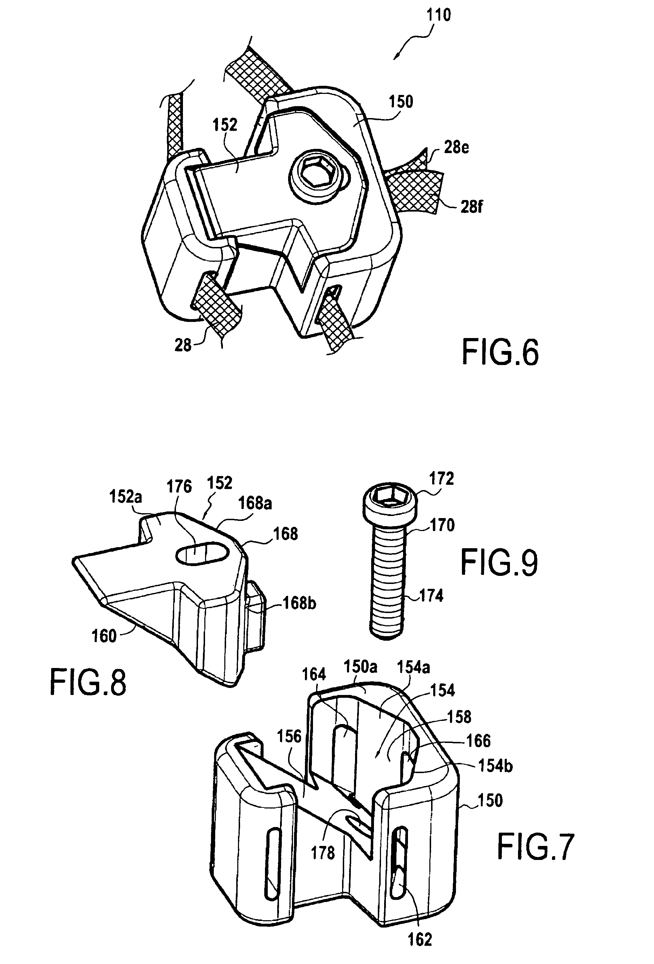

Other embodiments of spacers 110 are shown on Figures 6-17. These various embodiments differ essentially in the embodiment of the tie-securing system and, more particularly, of the moving part for clamping the end portions 28e and 28f of the tie 28.

Figures 6 to 9 show a spacer 110 quite similar to that shown in Figures 3 to 5. The spacer body 150 constitutes the stationary part of the securing system and the part 152 constitutes the moving part. The spacer body 150 is provided with a cavity 154, or housing, presenting a sloping end wall 156 and an inside front wall 158. The cavity 154 opens out into the top face 150a of the body 150. The moving part 152 has a bottom face 160 that can slide against the sloping end wall 156 of the cavity 154. As in the embodiment of Figures 3 to 5, the spacer body 150 has two side slots 162 and 164 and an axial slot 166 for passing the end portions 28e, 28f of the tie 28. The inside front face of the cavity 154 defines two clamping surfaces 154a and 154b. The moving part 152 presents a front face 168 that itself defines two clamping surfaces 168a and 168b suitable for co-operating with the clamping surfaces 154a and 154b of the spacer body 150.

As in the embodiment of Figures 3 to 5, by moving the moving part 152 in the cavity 154 relative to the stationary part 150, the pairs of clamping surfaces 154a & 168a, and 154b & 168b are moved towards or away from each other. This movement is preferably obtained with the help of a screw 170 whose head 172 bears against the top face 152a of the moving part 152 and whose threaded portion 174 passes freely through an oblong bore 176 formed in the moving part 152, and co-operates with tapping 178 formed in the end wall of the spacer body 150. By turning the screw 170, with its head 172 bearing against the top face 152a of the moving part 152, the moving part 152 is caused to move relative to the stationary part 150, thereby securing the tie 28 to the spacer 110.

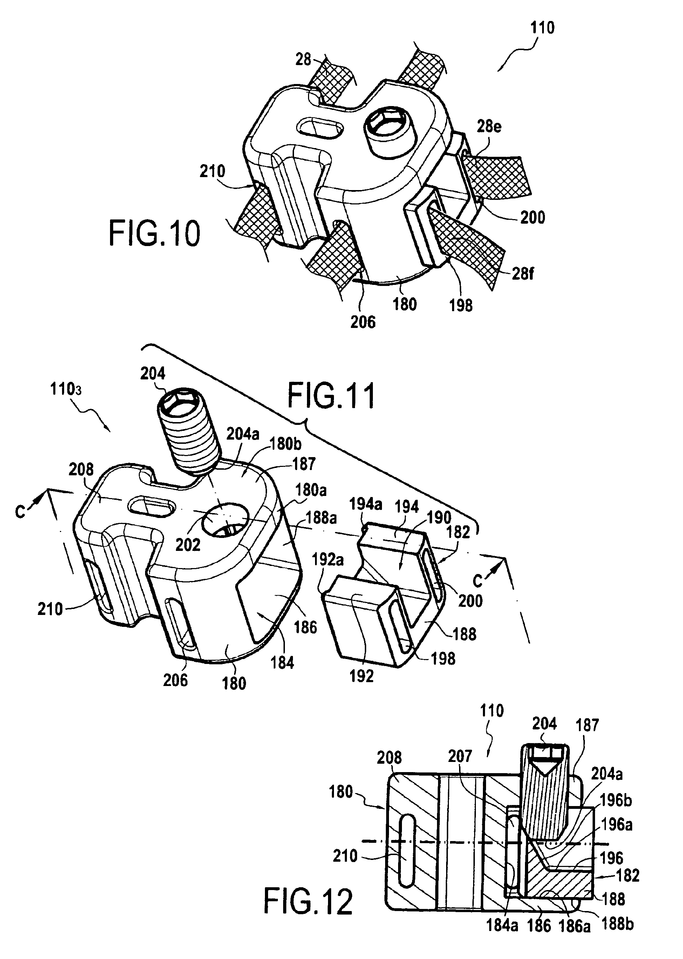

With reference to Figures 10 to 12, there follows a description of another embodiment of a spacer 110 fitted with a tie-securing system. Like the other spacers of Figures 3 to 7, the spacer 110 comprises a body 180 that constitutes the stationary part of the tie-securing system together with a moving part 182 that is mounted to slide in a cavity 184, or housing, formed in the body 180 of the spacer and opening out into the proximal face 180a of the spacer body 180. With reference to figure 12, the bottom of the cavity 184 is delimited by a substantially horizontal bottom wall 186 defining a sliding surface 186a on which the likewise sliding surface 182a of a substantially horizontal bottom wall 188 of the moving part 182 can slide.

The moving part 182 has, above its horizontal bottom wall 188, an axial channel 190 delimited between two flanges 192 and 194. As shown better in Figure 12, the bottom surface 196 of the axial channel 190 has a portion 196a that is substantially horizontal and a portion 196b that is inclined. The flanges 192 and 194 of the moving part 182 are provided with respective internal passages 198 and 200 for passing portions of the tie 28, said passages 198 and 200 extending parallel to the movement direction of the moving part 182. The spacer body 180 includes in its top wall 187 a tapped bore 202 suitable for co-operating with a screw 204. The bore 202 opens out into the housing 184. The spacer body 180 also has two side slots 206, 207 for passing portions of the tie 28, as shown more clearly in Figure 10. The end portions 28e, 28f of the tie 28 penetrate into the housing 184 via the side slots 206, 207 and pass through the slots 198 and 200 of the moving part 182 so that the end portions 28e and 28f of the tie 28 exit from the moving part 182, as shown in Figure 10. The distal faces 192a and 194a of the flanges 192 and 194 of the moving part 182 define clamping surfaces suitable for co-operating with the distal vertical clamping surface 184a of the housing 184 (see Figure 12).

The distal end 208 of the spacer 110 presents a free passage 210 for the tie 28.

As shown in Figure 12, the bottom end 204a of the screw 204 cooperates with the inclined portion 196b of the bottom surface 196 of the channel 190. Thus, by turning the screw 204, it is possible to move the moving part 182 in one direction or the other relative to the body 180. This serves to clamp portions of the tie 28 between the clamping surface 184a of the stationary part 180 and the clamping surfaces 192a and 194a of the moving part 182.

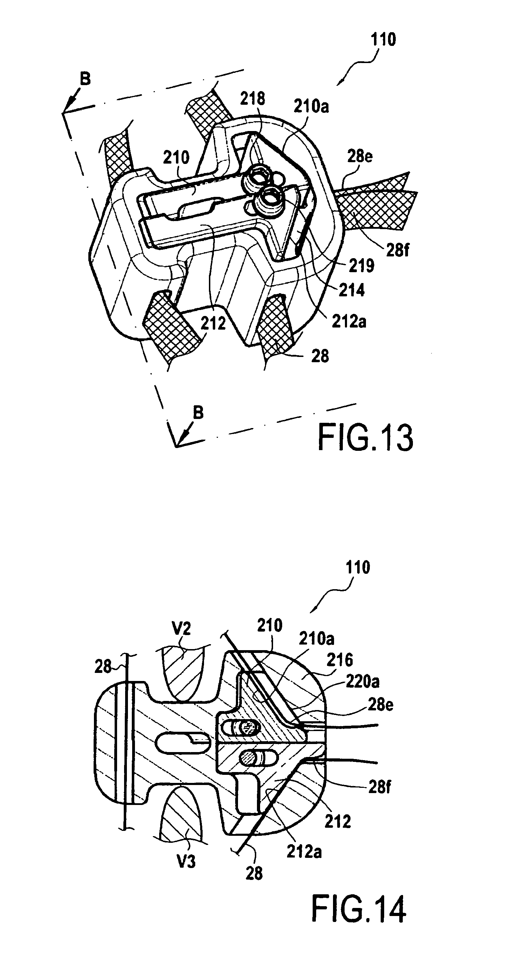

With reference to Figures 13 and 14, there follows a description of another embodiment of a spacer 110 fitted with a tie-securing system, this embodiment being quite similar to that of Figures 6 to 9. It differs therefrom essentially by the fact that the moving part is subdivided into two independent moving parts given respective references 210 and 212. These two moving parts 210 and 212 can be moved in translation inside the housing 214 that is formed in the spacer body 216. Each moving half-part 210 and 212 is fitted with a screw 218 and 219 for controlling its movement, each of these screws having exactly the same function as the screw 172 in the spacer 110 shown in Figures 6 to 9. The tie 28 is clamped and thus secured to the spacer 110 by co-operation between each of the clamping surfaces 210a and 212a as defined by the proximal ends of the moving half-parts 210 and 212 and by a common clamping surface 220a defined by a portion of the proximal wall of the body which delimits the housing 214. The advantage of this embodiment is that it enables each end portion 28e, 28f of the tie 28 to be secured separately to the spacer 110, which makes it possible under certain circumstances to enable the surgeon to perform special adjustment.

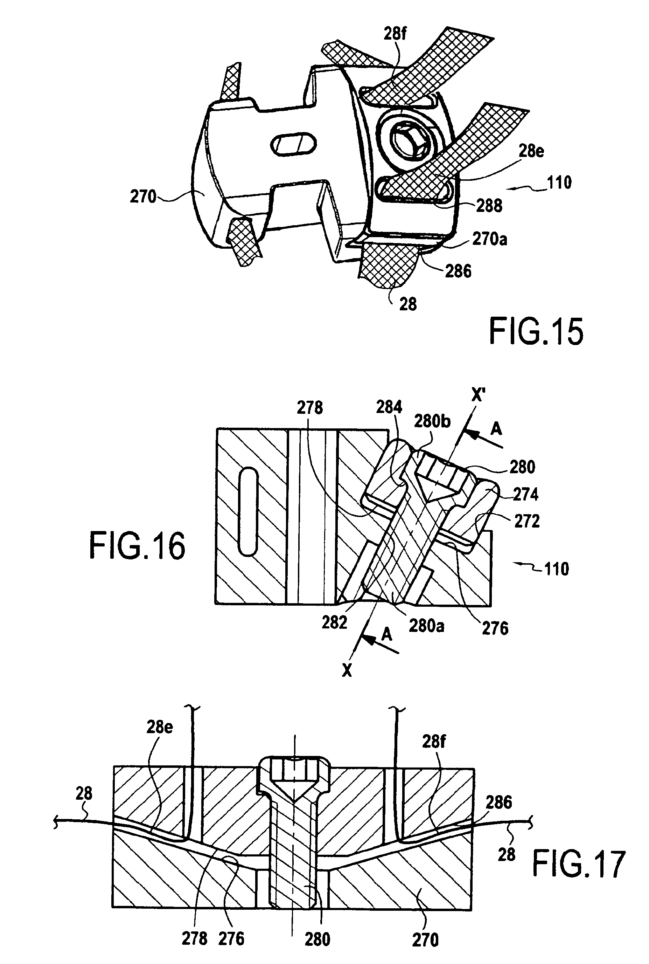

In Figures 15 to 17, there can be seen another embodiment of a spacer 110 that is provided with a securing system.

In this embodiment, the stationary part of the securing system is constituted by the spacer body 270. In the proximal part 270a of the spacer body there is provided a housing 272 for receiving a moving part 274. The bottom wall of the housing 272 constitutes a first clamping surface 276 for the tie 28. The bottom face of the moving part 274 constitutes a second clamping surface 278. The moving part 274 is moved in translation along the direction XX' (see Figure 16) by a control screw 280 having a threaded portion 280a that co-operates with tapping 282 formed in the spacer body 270. The head 280b of the screw 280 bears against the bottom of a counterbore 284 formed in the moving part 274.

End portions 28e, 28f of the tie 28 are inserted between the clamping surface 276 and 278 and pulled out of the moving part 274 via slots 288 formed in the moving part 274. By acting on the screw 280, the clamping surfaces 276 and 278 can be moved away from or towards each other.

It should be observed that the above-described tie-securing systems are all of the same general structure. They comprise two parts that are movable relative to each other and between which two free end portions 28e, 28f of the tie 28 are interposed. A mechanical member, usually a screw, serves to cause the parts to move towards or away from each other. When the two parts are moved towards each other, the two tie portions are secured to the spacer by being clamped or wedged.

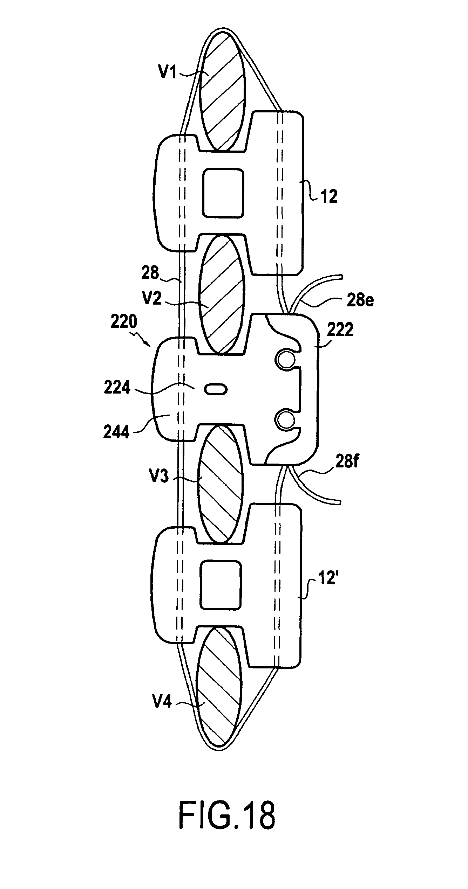

Figure 18 shows another embodiment of a stabilization system according to the present disclosure.

The stabilization system of Figure 18 differs from the stabilization system shown in Figure 2 by the fact that the spacer provided with a tie-securing system is of a different type.

The stabilization system as a whole comprises simple spacers 12, 12' interposed between vertebrae V1 and V2 and between vertebrae V3 and V4, and a tie 28 that co-operates with the spacers 12, 12', in exactly the same manner as in Figure 2. It further comprises a spacer 220 with a tie-securing system.

The spacer 220 differs essentially from the spacers 110 by the fact that the tie-securing system 222 that is associated therewith for securing the tie 28 with this spacer is removable from the body 224 of the spacer 220. More precisely, the tie-securing system is preferably secured to the spacer body 224 by a clip system as described below.

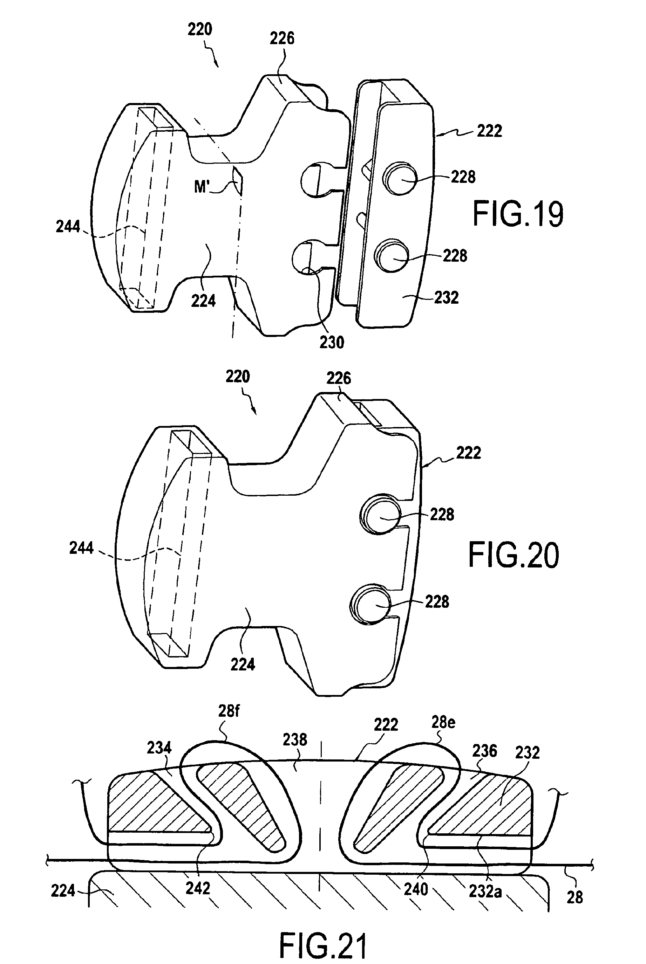

Figures 19 to 21 show the spacer 220 and its securing system 222. This spacer 220, which is preferably suitable for lateral insertion, has already been described in detail in PCT patent application WO 2007/099258. It is therefore described only briefly herein.

The securing system 222 can be fastened to the proximal end 226 of the spacer body 224 by a clip-fastener system that is constituted, for example, by two studs 228 carried by two side faces of the tie-securing system 222 suitable for co-operating with elastically deformable notches 230 formed in opposed side walls of the proximal end 226 of the spacer body 224. As shown more clearly in Figures 21, the tie 28 is secured to the spacer 220 by a system of slots made in the body 232 of the tie-securing system 222.

Such a tie-securing system 222 comprising two systems of slots that are self-locking is described in further details in PCT patent application WO 2007/120277. There are two inclined side slots 234 and 236 and an axial slot 238. The side slots 234 and 236 co-operate with the inside face 232a of the body 232 of the securing system 222 to define edges 240 and 242. Figure 21 shows the path followed by the tie 28 inside the body 232 of the securing system 222. The free end portions 28e and 28f of the tie 28 are inserted though the slots 234, 236, 238. Each free end portion 28e, 28f, of the tie is secured to the tie-securing system (and, thus, to the spacer 220) by locking the tie 28 because it follows a sinuous path through the system of slots, thereby ensuring that it becomes self-blocked by friction when a traction force is exerted on the middle portion of the tie 28.

When the securing system 222 has been clipped onto the spacer body 224 and when the surgeon is exerting appropriate traction on the free end portions 28e and 28f of the tie (generally using a surgical instrument), the tie 28 becomes wedged in the body 232 of the securing system, in particular because of the presence of the edges 240 and 242.

As the other spacers described above, the distal end of the body 224 of the spacer 220 has an internal passage 244 for receiving an intermediate portion of the tie 28. The internal passage 244 is located on the opposite side of the tie-securing system 222, with respect to the median plane M' of the spacer 220 (see figure 19).

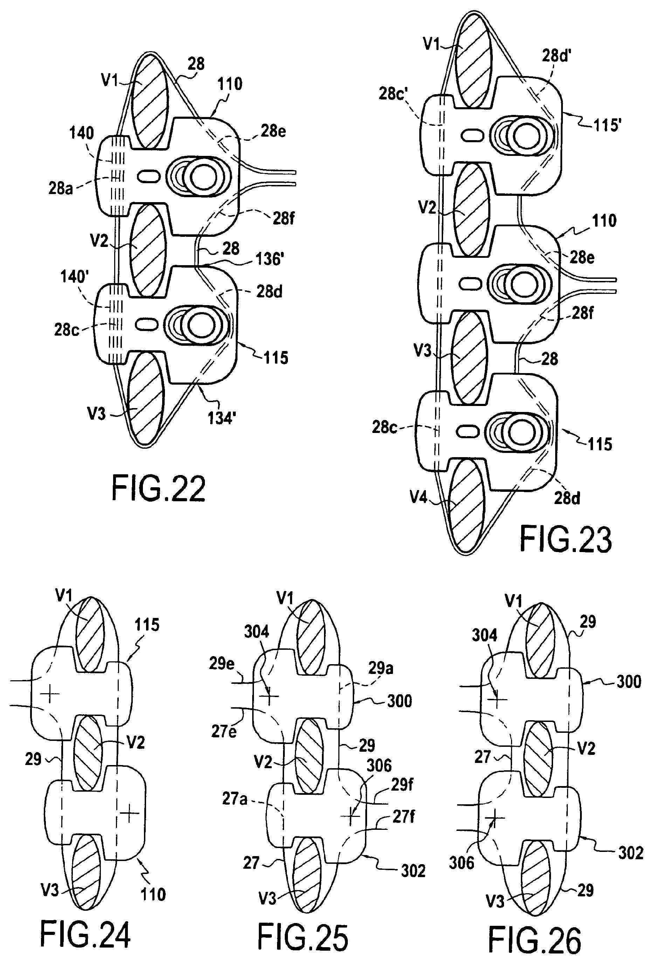

Figures 22 to 26 show other examples of stabilization systems according to the present disclosure.

The first example shown in Figure 22 is quite similar to the embodiment shown in Figure 1. The difference lies in the absence of simple spacer 12, the simple spacer 12 being replaced by a spacer 115 having a tie-securing system. The spacer 115 may have the same structure as the spacer 110, but it is used differently. The spacer 115 is not used to secure the free end portions 28e, 28f of the tie, but to guide one intermediate portion 28d of the tie. The tie 28 enters via the slot 134' (equivalent to slot 134 shown on Figures 3 and 4) and leaves via an analogous second slot 136' (equivalent to slot 136 shown on Figures 3 and 4).

Instead of having solely the function of guiding the intermediate portion 28d of the tie 28, the spacer 115 may also have the function of clamping this intermediate portion 28d. Consequently, the spacer 115 not only performs a guidance function, but it also performs a function of clamping an intermediate portion of the tie 28 by acting on the moving part (equivalent to the moving part 122 shown on Figures 4 and 5) of the spacer relative to its stationary part (equivalent to the stationary part 120 shown on Figures 4 and 5).

The spacer 115 further has an internal passage 140' (equivalent to the internal passage 140 shown on Figures 3 and 5) extending through the distal portion of its stationary part, for guiding another intermediate portion 28c of the tie 28.

Therefore, the spacer 115 has guiding means for guiding two intermediate portions 28c, 28d of the tie 28 and does not serve to secure the free end portions 28e, 28f of the tie 28, which latter function is performed by the spacer 110.

In Figure 23, there is shown another example of the stabilization system. In this example, there are three spacers 110, 115, 115'. Each one of these spacers has a tie-securing system, but only the tie-securing system of the spacer 110 is used to receive and secure the free end portions 28e, 28f of the tie 28. Each one of the spacers 115, 115' is used to guide two intermediate portions 28c, 28d, 28c', 28d' of the tie 28. Optionally, the spacer 115 and/or 115' may be used to clamp one 28d, 28d' of said two intermediate portions.

The spacers 110, 115 and 115' shown in Figures 22 and 23 are similar to the spacer 110 shown in Figures 3 to 5 but, of course, they could be similar to the other examples of spacers 110 shown in Figures 6 to 17.

Naturally, it would be possible to use a stabilization system comprising:
- a spacer 110 provided with a tie-securing system for securing the free end portions 28e, 28f, of the tie 28; and
- at least one simple spacer such as the spacer 12 shown in Figure 27 that serves solely to guide two intermediate portions of the tie 28, and/or at least one spacer such as the spacer 115 that serves to guide two intermediate portions of the tie 28 and, possibly, to clamp one of these two intermediate portions.

Figure 24 shows a variant of Figure 22 that differs therefrom by the fact that the spacers 110 and 115 are inserted between the spinous processes of the vertebrae V1, V2, and V3 using different lateral approaches. In other words, the spacer 110 is laterally inserted between the vertebrae V2 and V3 from one side of the vertebrae, whereas the spacer 115 is laterally inserted between the vertebrae V1 and V2 from the other side of the vertebrae. Therefore, the distal end of the spacer 115 is vertically aligned with the proximal end of the spacer 110.

Figure 25 shows another variant of the stabilization system according to the disclosure. In this variant, the stabilization system comprises two flexible ties 27, 29 adapted to form together a single loop around the spinous processes of three vertebrae V1, V2, V3. Each tie 27 (29) has two free end portions 27e, 27f (29e, 29f) and intermediate portions 27a (29a) therebetween. The stabilization system further comprises two spacers 300 and 302, each spacer 300 (302) being provided with:
- a tie-securing system 304 (306) suitable for securing one free end portion 27e, 29e (27f, 29f) of each of said two ties 27, 29, to the spacer 300 (302); and
- guiding means for guiding an intermediate portion 29a (27a) of one of said ties.

Thus, as shown in Figure 25, spacer 300 serves for securing the free end portion 27e of tie 27 and the free end portion 29e of tie 29, and for guiding the intermediate portion 29a of tie 29; and spacer 302 serves for securing the free end portion 27f of tie 27 and the free end portion 29f of tie 29, and for guiding the intermediate portion 27a of tie 27.

Spacers 300 and 302 may be similar to one of the examples of spacers 110 shown in Figures 3 to 17.

It is to be noted that one of the spacers 300, 302 may serve, at first, for securing the two ties 27 and 29 together while the other spacer may serve for securing the two remaining free end portions of the ties after the surgeon has exerted an appropriate amount of traction on these remaining free end portions, which exit through said other spacer.

This solution makes it possible, as a function of ease of access, to exert traction on the ties 27, 29, via one or the other of the spacers 300, 302 or to begin by exerting traction on the free end portions of the ties 27, 29, via one of the spacers and to finish off applying traction to the ties via the other spacer.

In Figure 25, the spacer 300 is laterally inserted between the vertebrae V1 and V2 from one side of the vertebrae, whereas the spacer 302 is laterally inserted between the vertebrae V2 and V3 from the other side of the vertebrae. In the variant of Figure 26, both spacers 300 and 302 are laterally inserted from the same side of the vertebrae V1, V2, V3. In this latter variant, one of the two ties 27, 29, is longer than the other.

## Claims

1. A stabilization system for stabilizing at least three vertebrae, comprising:
- a single flexible tie (28) having two free end portions (28e, 28f) and intermediate portions (28a, 28c, 28d) therebetween, said tie being adapted to form a single loop around the spinous processes of said vertebrae (V1, V2, V3); and
- a group of at least two spacers (12, 110), each spacer being adapted to be interposed between the spinous processes of two adjacent vertebrae, said group of spacers (12, 110) comprising at least:
- a first spacer (12) having guiding means for guiding two intermediate portions (28c, 28d) of the tie; and
- a second spacer (110) with a tie-securing system through which the two free end portions (28e, 28f) of the tie can be inserted and pulled, the tie-securing system allowing said free end portions (28e, 28f) to be fixedly secured to the second spacer (110).

2. A stabilization system according to claim 1, wherein said guiding means comprise two internal passages (24, 26) extending through the thickness of the first spacer (110) and located on either side of the median plane of the first spacer (110), said two intermediate portions (28c, 28d) passing, respectively, freely through the two internal passages (24, 26).

3. A stabilization system according to claim 1 or 2, wherein the first spacer (12) is a simple spacer not having any tie-securing system.

4. A stabilization system according to claim 1, wherein both the first and second spacers (110, 115) comprise a tie-securing system.

5. A stabilization system according to claim 4, wherein the first and second spacers (110, 115) are identical.

6. A stabilization system according to any one of claims 1 to 5, wherein the second spacer (110) has guiding means for guiding one intermediate portion (28a) of the tie.

7. A stabilization system according to claim 6, wherein said guiding means of the second spacer (110) comprise an internal passage (140) extending through the thickness of the second spacer and located on the opposite side of the tie-securing system, said intermediate portion (28a) passing freely through said internal passage (140).

8. A stabilization system according to any one of claims 1 to 7, wherein said tie-securing system comprises :
- two parts (120, 122) that are movable relative to each other, said parts (120, 122) defining clamping surfaces (130a, 130b, 132a, 132b) between which it is possible to place said free end portions (28e, 28f) of the tie, and
- a mechanical member (138) for causing said two parts (120, 122) to move towards each other, whereby the free end portions (28e, 28f) of the tie are clamped between said clamping surfaces (130a, 130b, 132a, 132b) and, thus, secured to the second spacer (110).

9. A system for stabilizing at least three vertebrae, comprising:
- at least two flexible ties (27, 29) adapted to form together a single loop around the spinous processes of said vertebrae (V1, V2, V3), each tie (27, 29) having two free end portions (27e, 27f, 29e, 29f) and intermediate portions (27a, 29a) therebetween; and
- at least two spacers (300, 302), each spacer being adapted to be interposed between the spinous processes of two adjacent vertebrae, and each spacer (300, 302) comprising:
- guiding means for guiding an intermediate portion (27a, 29a) of one of said ties (27, 29); and
- a tie-securing system (304, 306) through which one free end portion (27e, 29e, 27f, 29f) of each of said two ties (27, 29) can be inserted and pulled, the tie-securing system allowing these two free end portions (27e, 29e, 27f, 29f) to be fixedly secured to the spacer (300, 302).

10. A stabilization system according to claim 9, wherein said guiding means comprise an internal passage extending through the thickness of the spacer and located on the opposite side of the tie-securing system (304, 306), said intermediate portion (27a, 29a) passing freely through said internal passage.

11. A stabilization system according to claim 9 or 10, wherein said tie-securing system (304, 306) comprises :
- two parts that are movable relative to each other, said parts defining clamping surfaces between which it is possible to place said free end portions (27e, 29e, 27f, 29f) of the ties (27, 29), and
- a mechanical member for causing the two parts to move towards each other, whereby the free end portions of the ties are clamped between said clamping surfaces and, thus, secured to the spacer.

## Patentansprüche

1. Stabilisationssystem zum Stabilisieren von wenigstens drei Wirbeln, umfassend:
- ein einzelnes flexibles Bindeglied (28) mit zwei freien Endabschnitten (28e, 28f) und zwischenliegenden Abschnitten (28a, 28c, 28d) dazwischen, wobei das Bindeglied dazu geeignet ist, eine einzelne Schleife um die Dornfortsätze der Wirbel (V1, V2, V3) auszubilden, und
- eine Gruppe von wenigstens zwei Distanzstücken (12, 110), wobei jedes Distanzstück dazu geeignet ist, zwischen die Dornfortsätze von zwei benachbarten Wirbeln eingeschoben zu sein, wobei die Gruppe von Distanzstücken (12, 110) wenigstens umfaßt:
- ein erstes Distanzstück (12) mit Führungsmitteln zum Führen von zwei zwischenliegenden Abschnitten (28c, 28d) des Bindeglieds und
- ein zweites Distanzstück (110) mit einem Bindegliedsicherungssystem, durch das die zwei freien Endabschnitte (28e, 28f) des Bindeglieds eingeführt und gezogen sein können, wobei das Bindegliedsicherungssystem es den freien Endabschnitten (28e, 28f) erlaubt, an dem zweiten Distanzstück (110) fest gesichert zu sein.

2. Stabilisationssystem nach Anspruch 1, wobei die Führungsmittel zwei Innendurchgänge (24, 26) umfassen, die durch die Dicke des ersten Distanzstücks (110) verlaufen und sich auf jeder Seite der Mittelebene des ersten Distanzstücks (110) befinden, wobei zwei zwischenliegende Abschnitte (28c, 28d) die zwei Innendurchgänge (24, 26) jeweils frei durchlaufen.

3. Stabilisationssystem nach einem der Ansprüche 1 oder 2, wobei das erste Distanzstück (12) ein einfaches Distanzstück ist, das kein Bindegliedsicherungssystem aufweist.

4. Stabilisationssystem nach Anspruch 1, wobei das erste und zweite Distanzstück (110, 115) beide ein Bindegliedsicherungssystem umfassen.

5. Stabilisationssystem nach Anspruch 4, wobei das erste und zweite Distanzstück (110, 115) identisch sind.

6. Stabilisationssystem nach einem der Ansprüche 1 bis 5, wobei das zweite Distanzstück (110) Führungsmittel zum Führen von einem zwischenliegenden Abschnitt (28a) des Bindeglieds aufweist.

7. Stabilisationssystem nach Anspruch 6, wobei die Führungsmittel des zweiten Distanzstücks (110) einen Innendurchgang (140) umfassen, der durch die Dicke des zweiten Distanzstücks verläuft und sich auf der gegenüberliegenden Seite des Bindegliedsicherungssystems befindet, wobei der zwischenliegende Abschnitt (28a) den Innendurchgang (140) frei durchläuft.

8. Stabilisationssystem nach einem der Ansprüche 1 bis 7, wobei das Bindegliedsicherungssystem umfaßt:
- zwei Teile (120, 122), die relativ zueinander beweglich sind, wobei die Teile (120, 122) Einspannflächen (130a, 130b, 132a, 132b) definieren, zwischen denen es möglich ist, die freien Endabschnitte (28e, 28f) des Bindeglieds anzuordnen, und
- ein mechanisches Glied (138) zum Bewirken, daß sich die zwei Teile (120, 122) zueinander hin bewegen, wodurch die freien Endabschnitte (28e, 28f) des Bindeglieds zwischen den Einspannflächen (130a, 130b, 132a, 132b) eingespannt und dadurch an dem zweiten Distanzstück (110) gesichert sind.

9. System zum Stabilisieren von zumindest drei Wirbeln, umfassend:
- zumindest zwei flexible Bindeglieder (27, 29), die dazu geeignet sind, eine einzelne Schleife um die Dornfortsätze der Wirbel (V1, V2, V3) auszubilden, wobei jedes Bindeglied (27, 29) zwei freie Endabschnitte (27e, 27f, 29e, 29f) und zwischenliegende Abschnitte (27a, 29a) dazwischen aufweist, und
- zumindest zwei Distanzstücke (300, 302), wobei jedes Distanzstück dazu geeignet ist, zwischen die Dornfortsätze von zwei benachbarten Wirbeln eingeschoben zu sein, und wobei jedes Distanzstück (300, 302) umfaßt:
- Führungsmittel zum Führen eines zwischenliegenden Abschnitts (27a, 29a) von einem der Bindeglieder und
- ein Bindegliedsicherungssystem (304, 306), durch das ein freier Endabschnitt (27e, 29e, 27f, 29f) von jedem der zwei Bindeglieder (27, 29) eingeführt und gezogen sein kann, wobei das Bindegliedsicherungssystem es diesen zwei freien Endabschnitten (27e, 29e, 27f, 29f) erlaubt, an dem Distanzstück (300, 302) fest gesichert zu sein.

10. Stabilisationssystem nach Anspruch 9, wobei die Führungsmittel einen lnnendurchgang umfassen, der durch die Dicke des Distanzstücks verläuft und sich auf der gegenüberliegenden Seite des Bindegliedsicherungssystems befindet, wobei der zwischenliegende Abschnitt (27a, 29a) den Innendurchgang frei durchläuft.

11. Stabilisationssystem nach einem der Ansprüche 9 oder 10, wobei das Bindegliedsicherungssystem (304) umfaßt:
- zwei Teile, die in bezug zueinander beweglich sind, wobei die Teile Einspannflächen definieren, zwischen denen es möglich ist, die freien Endabschnitte (27e, 29e, 27f, 29f) der Bindeglieder (27, 29) anzuordnen, und
- ein mechanisches Glied zum Bewirken, daß sich die zwei Teile zueinander hin bewegen, wodurch die freien Endabschnitte der Bindeglieder zwischen den Einspannflächen eingespannt und dadurch an dem Distanzstück gesichert sind.

## Revendications

1. Système de stabilisation pour stabiliser au moins trois vertèbres, comprenant :
- un unique lien flexible (28) comprenant deux portions d'extrémité libre (28e, 28f) et des portions intermédiaires (28a, 28c, 28d) entre les portions d'extrémité libre, ce lien étant adapté pour former une unique boucle autour des apophyses épineuses desdites vertèbres (V1, V2, V3); et
- un groupe d'au moins deux cales (12, 110), chaque cale étant adaptée pour être interposée entre les apophyses épineuses de deux vertèbres adjacentes, ce groupe de cales (12, 110) comprenant au moins:
- une première cale (12) ayant des moyens de guidage pour guider deux portions intermédiaires (28c, 28d) du lien; et
- une deuxième cale (110) avec un système de solidarisation de lien à travers lequel les deux portions d'extrémité libre (28e, 28f) du lien peuvent être insérées et tirées, le système de solidarisation de lien permettant de solidariser fixement lesdites portions d'extrémité libre (28e, 28f) à la deuxième cale (110).

2. Système de stabilisation selon la revendication 1, dans lequel lesdits moyens de guidage comprennent deux passages internes (24, 26) s'étendant à travers l'épaisseur de la première cale (110) et situés de chaque côté du plan médian de la première cale (110), lesdites portions intermédiaires (28c, 28d) passant, respectivement, librement à travers les deux passages internes (24, 26).

3. Système de stabilisation selon la revendication 1 ou 2, dans lequel la première cale (12) est une cale simple n'ayant aucun système de solidarisation de lien.

4. Système de stabilisation selon la revendication 1, dans lequel les première et deuxième cales (110, 115) comprennent un système de solidarisation de lien.

5. Système de stabilisation selon la revendication 4, dans lequel les première et deuxième cales (110, 115) sont identiques.

6. Système de stabilisation selon l'une quelconque des revendications 1 à 5, dans lequel la deuxième cale (110) a des moyens de guidage pour guider une portion intermédiaire (28a) du lien.

7. Système de stabilisation selon la revendication 6, dans lequel les moyens de guidage de la deuxième cale (110) comprennent un passage interne (140) s'étendant à travers l'épaisseur de la deuxième cale et situé du côté opposé au système de solidarisation de lien, ladite portion intermédiaire (28a) passant librement à travers ledit passage interne (140).

8. Système de stabilisation selon l'une quelconque des revendications 1 à 7, dans lequel ledit système de solidarisation de lien comprend :
- deux pièces (120, 122) qui sont mobiles l'une par rapport à l'autre, lesdites pièces (120, 122) définissant des surfaces de serrage (130a, 130b, 132a, 132b) entre lesquelles il est possible de placer lesdites portions d'extrémité libre (28e, 28f) du lien, et
- un organe mécanique (138) pour provoquer le rapprochement des deux pièces (120, 138) l'une vers l'autre, par quoi les portions d'extrémité libre du lien sont serrées entre lesdites surfaces de serrage (130a, 130b, 132a, 132b) et, ainsi, solidarisées à la deuxième cale (110).

9. Système de stabilisation pour stabiliser au moins trois vertèbres, comprenant :
- au moins deux liens flexibles (27, 29) adaptés pour former ensemble une unique boucle autour des apophyses épineuses desdites vertèbres (V1, V2, V3), chaque lien (27, 29) ayant deux portions d'extrémité libre (27e, 27f, 29e, 29f) et des portions intermédiaires (27a, 29a) entre ces portions d'extrémité libre; et
- au moins deux cales (300, 302), chaque cale étant adaptée pour être interposée entre les apophyses épineuses de deux vertèbres adjacentes, chaque cale (300, 302) comprenant:
- des moyens de guidage pour guider une portion intermédiaire (27a, 29a) d'un desdits liens (27, 29) ; et
- un système de solidarisation de lien (304, 306) à travers lequel une portion d'extrémité libre (27e, 27f, 29e, 29f) de chacun desdits liens (27, 29) peut être insérée et tirée, le système de solidarisation de lien permettant de solidariser fixement ces portions d'extrémité libre (27e, 27f, 29e, 29f) à la cale (300, 302).

10. Système de stabilisation selon la revendication 9, dans lequel les moyens de guidage comprennent un passage interne s'étendant à travers l'épaisseur de la deuxième cale et situé du côté opposé au système de solidarisation de lien (304, 306), ladite portion intermédiaire (27a, 29a) passant librement à travers ledit passage interne.

11. Système de stabilisation selon la revendication 9 ou 10, dans lequel ledit système de solidarisation de lien (304, 306) comprend :
- deux pièces qui sont mobiles l'une par rapport à l'autre, lesdites pièces définissant des surfaces de serrage entre lesquelles il est possible de placer lesdites portions d'extrémité libre (27e, 27f, 29e, 29f) des liens (27, 29), et
- un organe mécanique pour provoquer le rapprochement des deux pièces l'une vers l'autre, par quoi les portions d'extrémité libre du lien sont serrées entre lesdites surfaces de serrage et, ainsi, solidarisées à la cale.
